# EUROPEAN PATENT APPLICATION

(11) **EP 1 972 344 A1**
(43) Date of publication of application: **24.09.2008**
(21) Application number: 08006597.2
(22) Date of filing: 22.12.2003
(51) Int. Cl.: A61K 38/00, A61K 47/00, A61P 39/04

(54) **Ophthalmic formulation for the prevention and treatment of ocular conditions**

(30) Priority: 20.12.2002 US 435849 P; 26.09.2003 US 506474 P
(62) Divisional of application: 03814363.2
(71) Applicant: Chakshu Research, Inc., Los Gatos, CA 95030 (US)
(72) Inventor: BHUSHAN, Rajiv, Palo Alto, CA 94306 (US)
(74) Representative: Mallalieu, Catherine Louise

(57) **Abstract**

An ophthalmic formulation is provided for the prevention and treatment of adverse ocular conditions, including presbyopia, arcus senilis, age-related macular degeneration, and other conditions associated with aging. The formulation is also useful in the prevention and treatment of other adverse ocular conditions such as those associated with oxidative and/or free radical damage within the eye; these conditions can involve a condition, disease, or disorder of the cornea, retina, lens, sclera, anterior segment, or posterior segment of the eye. In one embodiment, the formulation contains at least 0.6 wt.% of a biocompatible chelating agent, an effective permeation enhancing amount of an ophthalmic permeation enhancer such as methylsulfonylmethane (MSM), an anti-AGE agent, i.e., a compound that serves to reduce the presence of advanced glycation endproducts (AGEs) in the eye, and a pharmaceutically acceptable ophthalmic carrier suited to the particular formulation type (e.g., eye drops or ointments). Preferred components of the formulation are multifunctional and naturally occurring.

## Description

### TECHNICAL FIELD

This invention relates generally to the treatment of ocular disorders, ocular diseases, and other adverse ocular conditions. More particularly, the invention pertains to an ophthalmic formulation for the prevention and treatment of various adverse ocular conditions, including those associated with aging. The invention also pertains to the use of the formulation in improving vision and the cosmetic appearance of the eye. Accordingly, the invention finds utility in a variety of fields, including ophthalmology, geriatrics, and cosmeceutics.

### BACKGROUND ART

Progressive, age-related changes of the eye, including normal as well as pathological changes, have always been an unwelcome but inevitable part of extended life in humans and other mammals. Many of these changes seriously affect both the function and the cosmetic appearance of the eyes. These changes include: development of cataracts; hardening, opacification, reduction of pliability, and yellowing of the lens; yellowing and opacification of the cornea; presbyopia; clogging of the trabeculum, leading to intraocular pressure build-up and glaucoma; increased floaters in the vitreous humor; stiffening and reduction of the dilation range of the iris; age-related macular degeneration (AMD); formation of atherosclerotic deposits in retinal arteries; dry eye syndrome; and decreased sensitivity and light level adaptation ability of the rods and cones of the retina. Age-related vision deterioration includes loss in visual acuity, visual contrast, color and depth perception, lens accommodation, light sensitivity, and dark adaptation. Age-related changes also include changes in the color appearance of the iris, and formation of arcus senilis. The invention is, in large part, directed toward a formulation and method for preventing and treating a multiplicity of age-related ocular disorders and diseases.

All parts of the eye, including the cornea, sclera, trabeculum, iris, lens, vitreous humor, and retina are affected by the aging process, as explained below.

### The Cornea:

The cornea is the eye's outermost layer. It is the clear, dome-shaped surface that covers the front of the eye. The cornea is composed of five layers. The epithelium is a layer of cells that forms the surface. It is only about 5-6 cell layers thick and quickly regenerates when the cornea is injured. If an injury penetrates more deeply into the cornea, scarring may occur and leave opaque areas, causing the cornea to lose its clarity and luster. Immediately below the epithelium is Bowman's membrane, a protective layer that is very tough and difficult to penetrate. The stroma, the thickest layer of the cornea, lies just beneath Bowman's membrane and is composed of tiny collagen fibrils aligned in parallel, an arrangement that provides the cornea with its clarity. Descemet's membrane underlies the stroma and is just above the innermost corneal layer, the endothelium. The endothelium is just one cell layer in thickness, and serves to pump water from the cornea to the aqueous, keeping it clear. If damaged or diseased, these cells will not regenerate.

As the eye ages, the cornea can become more opaque. Opacification can take many forms. The most common form of opacification affects the periphery of the cornea, and is termed "arcus senilis," or "arcus." This type of opacification initially involves deposition of lipids into Descemet's membrane. Subsequently, lipids deposit into Bowman's membrane and possibly into the stroma as well. Arcus senilis is usually not visually significant, but is a cosmetically noticeable sign of aging. There are other age related corneal opacifications, however, which may have some visual consequences. These include central cloudy dystrophy of Francois, which affects the middle layers of the stroma, and posterior crocodile shagreen, which is central opacification of the posterior stroma. Opacification, by scattering light, results in progressive reduction of visual contrast and visual acuity.

Opacification of the cornea develops as a result of a number of factors, including, by way of example: degeneration of corneal structure; cross-linking of collagen and other proteins by metalloproteinases; ultraviolet (UV) light damage; oxidation damage; and buildup of substances like calcium salts, protein waste, and excess lipids.

There is no established treatment for slowing or reversing corneal changes other than surgical intervention. For example, opaque structures can be scraped away with a blunt instrument after first removing the epithelium, followed by smoothing and sculpting the corneal surface with a laser beam. In severe cases of corneal scarring and opacification, corneal transplantation has been the only effective approach.

Another common ocular disorder that adversely affects the cornea as well as other structures within the eye is keratoconjunctivitis sicca, commonly referred to as "dry eye syndrome" or "dry eye." Dry eye can result from a host of causes, and is frequently a problem for older people. The disorder is associated with a scratchy sensation, excessive secretion of mucus, a burning sensation, increased sensitivity to light, and pain. Dry eye is currently treated with "artificial tears," a commercially available product containing a lubricant such as low molecular weight polyethylene glycol. Surgical treatment, also, is not uncommon, and usually involves insertion of a punctal plug so that lacrimal secretions are retained in the eye. However, both types of treatment are problematic: surgical treatment is invasive and potentially risky, while artifical tear products provide only very temporary and often inadequate relief.

### The sclera:

The sclera is the white of the eye. In younger individuals, the sclera has a bluish tinge, but as people grow older, the sclera yellows as a result of age-related changes in the conjunctiva. Over time, UV and dust exposure may result in changes in the conjunctival tissue, leading to pingecula and pterygium formation. These ocular growths can further cause breakdown of scleral and corneal tissue. Currently, surgery, including conjunctival transplantation, is the only accepted treatment for pingeculae and pterygia.

### The trabeculum:

The trabeculum, also referred to as the trabecular meshwork, is a mesh-like structure located at the iris-sclera junction in the anterior chamber of the eye. The trabeculum serves to filter aqueous fluid and control its flow from the anterior chamber into the canal of Schlemm. As the eye ages, debris and protein-lipid waste may build up and clog the trabeculum, a problem that results in increased pressure within the eye, which in turn can lead to glaucoma and damage to the retina, optic nerve, and other structures of the eye. Glaucoma drugs can help reduce this pressure, and surgery can create an artificial opening to bypass the trabeculum and reestablish flow of liquid out of the vitreous and aqueous humor. There is, however, no known method for preventing a build-up of debris and protein-lipid waste within the trabeculum.

### The iris and pupil:

With age, dilation and constriction of the iris in response to changes in illumination become slower, and its range of motion decreases. Also, the pupil becomes progressively smaller with age, severely restricting the amount of light entering the eye, especially under low light conditions. The narrowing pupil and the stiffening, slower adaptation, and constriction of the iris over time are largely responsible for the difficulty the aged have in seeing at night and adapting to changes in illumination. The changes in iris shape, stiffness, and adaptability are generally thought to come from fibrosis and cross-linking between structural proteins. Deposits of protein and lipid wastes on the iris over time may also lighten its coloration. Both the light-colored deposits on the iris, and narrowing of the pupil, are very noticeable cosmetic markers of age that may have social implications for individuals. There is no standard treatment for any of these changes, or for changes in iris coloration with age.

### The Lens:

With age, the lens yellows, becomes harder, stiffer, and less pliable, and can opacify either diffusely or in specific locations. Thus, the lens passes less light, which reduces visual contrast and acuity. Yellowing also affects color perception. Stiffening of the lens as well as the inability of the muscle to accommodate the lens results in a condition generally known as presbyopia. Presbyopia, almost always occurring after middle age, is the inability of an eye to focus correctly. This age-related ocular pathology manifests itself in a loss of accommodative ability, i.e., the capacity of the eye, through the lens, to focus on near or far objects by changing the shape of the lens to become more spherical (or convex). Both myopic and hyperopic individuals are subject to presbyopia. The age-related loss of accommodative amplitude is progressive, and presbyopia is perhaps the most prevalent of all ocular afflictions, ultimately affecting virtually all individuals during the normal human life span.

These changes in the lens are thought to be due to degenerative changes in the structure of the lens, including glycated crosslinks between collagen fibers, buildup of protein complexes, ultraviolet light degradation of structures, oxidation damage, and deposits of waste proteins, lipids, and calcium salts. Elastic and viscous properties of the lens are dependent on properties of the fiber membranes and cytoskeleton crystallins. The lens fiber membranes are characterized by an extremely high cholesterol to phospholipid ratio. Any changes in these components affect the deformability of the lens membrane. The loss of lens deformability has also been attributed to increased binding of lens proteins to the cell membranes.

Compensatory options to alleviate presbyopia currently include bifocal reading glasses and/or contact lenses, monovision intraocular lenses (IOLs) and/or contact lenses, multifocal IOLs, monovision and anisometropic corneal refractive surgical procedures using radial keratotomy (RK), photorefractive keratomileusis (PRK), and laser-assisted in situ keratomileusis (LASIK). No universally accepted treatments or cures are currently available for presbyopia.

Opacity of the lens results in an abnormal condition generally known as cataract. Cataract is a progressive ocular disease, which subsequently leads to lower vision. Most of this ocular disease is age-related senile cataract. The incidence of cataract formation is thought to be 60-70% in persons in their sixties and nearly 100% in persons eighty years or older. However, at the present time, there is no agent that has been clearly proven to inhibit the development of cataracts. Therefore, the development of an effective therapeutic agent has been desired. Presently, the treatment of cataracts depends upon the correction of vision using eyeglasses, contact lenses, or surgical operations such as insertion of an intra-ocular lens into the capsula lentis after extra-capsular cataract extraction.

In cataract surgery, the incidence of secondary cataract after surgery has been a problem. Secondary cataract is equated with opacity present on the surface of the remaining posterior capsule following extracapsular cataract extraction. The mechanism of secondary cataract is mainly as follows. After excising lens epithelial cells (anterior capsule), secondary cataract results from migration and proliferation of residual lens epithelial cells, which are not completely removed at the time of extraction of the lens cortex, onto the posterior capsule leading to posterior capsule opacification. In cataract surgery, it is impossible to remove lens epithelial cells completely, and consequently it is difficult to always prevent secondary cataract. It is said that the incidence of the above posterior capsule opacification is 40-50% in eyes that do not receive an intracapsular posterior chamber lens implant and 7-20% in eyes which do receive an intracapsular lens implant. Additionally, eye infections categorized as endophthalmitis have also been observed after cataract surgeries.

### The vitreous humor:

Floaters are debris particles that interfere with clear vision by projecting shadows on the retina. There currently is no standard treatment for reducing or eliminating floaters.

### The retina:

A number of changes can occur in the retina with age. Atherosclerotic buildup and leakage in the retinal arteries can lead to macular degeneration as well as reduction of peripheral vision. The rods and cones can become less sensitive over time as they replenish their pigments more slowly. Progressively, all these effects can reduce vision, ultimately leading to partial or complete blindness. Retinal diseases such as age-related macular degeneration have been hard to cure. Current retinal treatments include laser surgery to stop the leaking of blood vessels in the eye.

As alluded to above, current therapeutic attempts to address many ocular disorders and diseases, including aging-related ocular problems, often involve surgical intervention. Surgical procedures are, of course, invasive, and, furthermore, often do not achieve the desired therapeutic goal. Additionally, surgery can be very expensive and may result in significant undesired after-effects. For example, secondary cataracts may develop after cataract surgery and infections may set in. Endophthalmitis has also been observed after cataract surgery. In addition, advanced surgical techniques are not universally available, because they require a very well developed medical infrastructure. Therefore, it would be of significant advantage to provide straightforward and effective pharmacological therapies that obviate the need for surgery.

There have been products proposed to address specific, individual aging-related ocular conditions. For example, artificial tears and herbal formulations such as Simalasan eyedrops have been suggested for treating dry eye syndrome, and other eyedrops are available to reduce intraocular pressure, alleviate discomfort, promote healing after injury, reduce inflammation, and prevent infections. However, self-administration of multiple products several times a day is inconvenient, potentially results in poor patient compliance (in turn reducing overall efficacy), and can involve detrimental interaction of formulation components. For example, the common preservative benzalkonium chloride may react with other desirable components such as ethylenediamine tetraacetic acid (EDTA). Accordingly, there is a need in the art for a comprehensive pharmaceutical formulation that can prevent, arrest, and/or reverse a multiplicity of aging-related vision problems and the associated ocular disorders.

To date, such a formulation has not been provided, in large part because complex, multi-component pharmaceutical products are often problematic for formulators and manufacturers. Problems can arise, for example, from combining agents having different solubility profiles and/or membrane transport rates. With respect to the latter consideration, transport facilitators, also termed "permeation enhancers," need to be incorporated into a formulation, and must be pharmaceutically acceptable, have no effect on formulation stability, and be inert to and compatible with other components of the formulation and the physiological structures with which the formulation will come into contact.

### DISCLOSURE OF THE INVENTION

The present invention is directed to the aforementioned need in the art, and, in one embodiment, provides a sterile ophthalmic formulation containing:

a biocompatible chelating agent at a concentration of at least 0.6% by weight;

an effective permeation-enhancing concentration of a permeation enhancer;

an agent suitable for reducing the presence of Advanced Glycation Endproducts (AGE), i.e., an anti-AGE agent, selected from AGE breakers, AGE formation inhibitors, and glycation inhibitors; and

a pharmaceutically acceptable ophthalmic carrier.

In another embodiment, the invention provides a sterile ophthalmic formulation containing:

a biocompatible chelating agent at a concentration of at least 0.6% by weight;

an effective permeation-enhancing amount of methylsulfonylmethane; and

a pharmaceutically acceptable ophthalmic carrier.

In an additional embodiment, the invention provides a sterile ophthalmic formulation containing:

a biocompatible chelating agent at a concentration of at least 0.6% by weight;

an effective AGE-reducing concentration of L-carnosine; and

a pharmaceutically acceptable ophthalmic carrier.

The ophthalmic formulation may be administered in any form suitable for ocular drug administration, e.g., as a solution, suspension, ointment, gel, liposomal dispersion, colloidal microparticle suspension, or the like, or in an ocular insert, e.g., in an optionally biodegradable controlled release polymeric matrix. Significantly, at least one component of the formulation, and preferably two or more formulation components, are "multifunctional" in that they are useful in preventing or treating multiple conditions and disorders, or have more than one mechanism of action, or both. Accordingly, the present formulations eliminate a significant problem in the art, namely, cross-reaction between different formulation types and/or active agents when multiple formulations are used to treat a patient with multiple ocular disorders. Additionally, in a preferred embodiment, the formulation is entirely composed of components that are naturally occurring and/or as GRAS ("Generally Regarded as Safe") by the U.S. Food and Drug Administration.

The invention also pertains to methods of using the inventive formulation in the prevention and treatment of adverse ocular conditions, generally although not necessarily involving oxidative and/or free radical damage in the eye, and including, by way of example, conditions, diseases, or disorders of the cornea, retina, lens, sclera, and anterior and posterior segments of the eye. An adverse ocular condition as that term is used herein may be a "normal" condition that is frequently seen in aging individuals (e.g., decreased visual acuity and contrast sensitivity) or a pathologic condition that may or may not be associated with the aging process. The latter adverse ocular conditions include a wide variety of ocular disorders and diseases. Aging-related ocular problems that can be prevented and/or treated using the present formulations include, without limitation, opacification (both corneal and lens opacification), cataract formation (including secondary cataract formation) and other problems associated with deposition of lipids, visual acuity impairment, decreased contrast sensitivity, photophobia, glare, dry eye, loss of night vision, narrowing of the pupil, presbyopia, age-related macular degeneration, elevated intraocular pressure, glaucoma, and arcus senilis. By "aging-related" is meant a condition that is generally recognized as occurring far more frequently in older patients, but that may and occasionally do occur in younger people. The formulations can also be used in the treatment of ocular surface growths such as pingueculae and pterygia, which are typically caused by dust, wind, or ultraviolet light, but may also be symptoms of degenerative diseases associated with the aging eye. Another adverse condition that is generally not viewed as aging-related but which can be treated using the present formulation includes keratoconus. It should also be emphasized that the present formulation can be advantageously employed to improve visual acuity, in general, in any mammalian individual. That is, ocular administration of the formulation can improve visual acuity and contrast sensitivity as well as color and depth perception regardless of the patient's age or the presence of any adverse ocular conditions.

The invention also pertains to ocular inserts for the controlled release of a biocompatible chelating agent as noted above, e.g., EDTA, and/or an anti-AGE agent such as L-carnosine. The insert may be a gradually but completely soluble implant, such as may be made by incorporating swellable, hydrogel-forming polymers into an aqueous liquid formulation. The insert may also be insoluble, in which case the agent is released from an internal reservoir through an outer membrane via diffusion or osmosis.

### DETAILED DESCRIPTION OF THE DRAWINGS

FIGS. 1A, 1B, 2A, and 2B are photographs of the eyes of a 46-year-old male subject prior to treatment (OD - FIG. 1A; OS-FIG. 2A) and after (OS - FIG. 1B; and OS-FIG. 2B) receiving eight weeks of treatment with an eye drop formulation of the invention.

FIGS. 3A, 3B, 4A, and 4B are photographs of the eyes of a 60-year-old male subject prior to treatment (OD - FIG. 3A; OS-FIG. 4A) and after (OS - FIG. 3B; and OS-FIG. 3B) receiving eight weeks of treatment with an eye drop formulation of the invention.

### DETAILED DESCRIPTION OF THE PREFERRED EMBODIMENTS

### L. DEFINITIONS AND NOMENCLATURE

Unless otherwise indicated, the invention is not limited to specific formulation types, formulation components, dosage regimens, or the like, as such may vary. It is also to be understood that the terminology used herein is for the purpose of describing particular embodiments only and is not intended to be limiting.

As used in the specification and the appended claims, the singular forms "a," "an," and "the" include plural referents unless the context clearly dictates otherwise. Thus, for example, reference to "an anti-AGE agent" includes a single such agent as well as a combination or mixture of two or more different anti-AGE agents, reference to "a permeation enhancer" includes not only a single permeation enhancer but also a combination or mixture of two or more different permeation enhancers, reference to "a pharmaceutically acceptable ophthalmic carrier" includes two or more such carriers as well as a single carrier, and the like.

In this specification and in the claims that follow, reference will be made to a number of terms, which shall be defined to have the following meanings:

When referring to a formulation component, it is intended that the term used, e.g., "agent," encompass not only the specified molecular entity but also its pharmaceutically acceptable analogs, including, but not limited to, salts, esters, amides, prodrugs, conjugates, active metabolites, and other such derivatives, analogs, and related compounds.

The terms "treating" and "treatment" as used herein refer to the administration of an agent or formulation to a clinically symptomatic individual afflicted with an adverse condition, disorder, or disease, so as to effect a reduction in severity and/or frequency of symptoms, eliminate the symptoms and/or their underlying cause, and/or facilitate improvement or remediation of damage. The terms "preventing" and "prevention" refer to the administration of an agent or composition to a clinically asymptomatic individual who is susceptible to a particular adverse condition, disorder, or disease, and thus relates to the prevention of the occurrence of symptoms and/or their underlying cause. Unless otherwise indicated herein, either explicitly or by implication, if the term "treatment" (or "treating") is used without reference to possible prevention, it is intended that prevention be encompassed as well, such that "a method for the treatment of presbyopia" would be interpreted as encompassing "a method for the prevention of presbyopia."

By the terms "effective amount" and "therapeutically effective amount" of a formulation or formulation component is meant a nontoxic but sufficient amount of the formulation or component to provide the desired effect.

The term "controlled release" refers to an agent-containing formulation or fraction thereof in which release of the agent is not immediate, i.e., with a "controlled release" formulation, administration does not result in immediate release of the agent into an absorption pool. The term is used interchangeably with "nonimmediate release" as defined in Remington: The Science and Practice of Pharmacy, Nineteenth Ed (Easton, PA: Mack Publishing Company, 1995). In general, the term "controlled release" as used herein refers to "sustained release" rather than to "delayed release" formulations. The term "sustained release" (synonymous with "extended release") is used in its conventional sense to refer to a formulation that provides for gradual release of an agent over an extended period of time.

By "pharmaceutically acceptable" is meant a component that is not biologically or otherwise undesirable, i.e., the component may be incorporated into an ophthalmic formulation of the invention and administered topically to a patient's eye without causing any undesirable biological effects or interacting in a deleterious manner with any of the other components of the formulation composition in which it is contained. When the term "pharmaceutically acceptable" is used to refer to a component other than a pharmacologically active agent, it is implied that the component has met the required standards of toxicological and manufacturing testing or that it is included on the Inactive Ingredient Guide prepared by the U.S. Food and Drug Administration.

In one embodiment, an ophthalmic formulation is provided that comprises, in sterilized form, an admixture of: a biocompatible chelating agent at a concentration of at least 0.6% by weight; an effective permeation-enhancing concentration of a permeation enhancer; an anti-AGE agent selected from AGE breakers, AGE formation inhibitors, and glycation inhibitors; and a pharmaceutically acceptable ophthalmic carrier. The formulation may be applied to the eye in any form suitable for ocular drug administration, e.g., as a solution or suspension for administration as eye drops or eye washes, as an ointment, or in an ocular insert that can be implanted in the conjunctiva, sclera, pars plana, anterior segment, or posterior segment of the eye. Implants provide for controlled release of the formulation to the ocular surface, typically sustained release over an extended time period.

The biocompatible chelating agent is a sequestrant of divalent or polyvalent metal cations, and generally represents about 0.6 wt.% to 10 wt.%, preferably about 1.0 wt.% to 5.0 wt.%, of the formulation. The invention is not limited with regard to specific biocompatible chelating agents, and any biocompatible chelating agent can be used providing that it is capable of being buffered to a pH in the range of about 6.5 to about 8.0 and does not interact with any other component of the formulation. Suitable biocompatible chelating agents useful in conjunction with the present invention include, without limitation, monomeric polyacids such as EDTA, cyclohexanediamine tetraacetic acid (CDTA), hydroxyethylethylenediamine triacetic acid (HEDTA), diethylenetriamine pentaacetic acid (DTPA), dimercaptopropane sulfonic acid (DMPS), dimercaptosuccinic acid (DMSA), aminotrimethylene phosphonic acid (ATPA), citric acid, ophthalmologically acceptable salts thereof, and combinations of any of the foregoing. Other exemplary chelating agents include: phosphates, e.g., pyrophosphates, tripolyphosphates, and, hexametaphosphates; chelating antibiotics such as chloroquine and tetracycline; nitrogen-containing chelating agents containing two or more chelating nitrogen atoms within an imino group or in an aromatic ring (e.g., diimines, 2,2'-bipyridines, etc.); and polyamines such as cyclam (1,4,7,11-tetraazacyclotetradecane), N-(C₁-C₃₀ alkyl)-substituted cyclams (e.g., hexadecyclam, tetramethylhexadecylcyclam), diethylenetriamine (DETA), spermine, diethylnorspermine (DENSPM), diethylhomo-spermine (DEHOP), and deferoxamine (N'-[5-[[4-[[5-(acetylhydroxyamino)pentyl]amino]-1,4-dioxobutyl]hydroxyamino]pentyl]-N'-(5-aminopentyl)-*N*-hydroxybutanediamide; also known as desferrioxamine B and DFO).

EDTA and ophthalmologically acceptable EDTA salts are particularly preferred, wherein representative ophthalmologically acceptable EDTA salts are typically selected from diammonium EDTA, disodium EDTA, dipotassium EDTA, triammonium EDTA, trisodium EDTA, tripotassium EDTA, and calcium disodium EDTA.

EDTA has been widely used as an agent for chelating metals in biological tissue and blood, and has been suggested for inclusion in ophthalmic formulations. For example, U.S. Patent No. 5,817,630 to Hofmann et al. describes the incorporation of 0.05 wt.% to 0.5 wt.% EDTA into glutathione eye drops, U.S. Patent No. 5,283,236 to Chiou describes the use of EDTA as a permeation-enhancing agent for the systemic delivery of polypeptides through the eye, U.S. Patent No. 6,376,534 to Isaji et al. suggests that EDTA may be effective in inhibiting secondary cataracts, and U.S. Patent No. 6,348,508 to Denick Jr. et al. describes EDTA as a sequestering agent to bind metal ions. In addition to its use as a chelating agent, EDTA has also been widely used as a preservative in place of benzalkonium chloride, as described, for example, in U.S. Patent No. 6,211,238 to Castillo et al. U.S. Patent No. 6,265,444 to Bowman et al. discloses use of EDTA as a preservative and stabilizer.
However, EDTA has generally not been applied topically in any significant concentration in ophthalmic formulations because of its poor penetration through the epithelium of the cornea.

Without wishing to be bound by theory, it appears that a significant role played by the biocompatible chelating agent in the present formulations is in the removal of the active sites of metalloproteinases in the eye by sequestration of the enzymes' metal center. By inactivating metalloproteinases in this way, the chelating agent may slow or stop the degeneration of protein complexes within the eye, thereby providing an opportunity for the ocular tissues to rebuild themselves. In addition, by chelating metal ions such as copper, iron, and calcium, which are critical to the formation and proliferation of free radicals in the eye, the chelating agent forms complexes that are flushed into the bloodstream and excreted renally. In this way, the production of oxygen free radicals and reactive molecular fragments is reduced, in turn reducing pathological lipid peroxidation of cell membranes, DNA, enzymes, and lipoproteins, allowing the body's natural healing mechanisms to halt and reverse disease processes in progress.

Accordingly, the chelating agent is multifunctional in the context of the present invention, insofar as the agent serves to decrease unwanted proteinase (e.g., collagenase) activity, prevent formation of lipid deposits, and/or reduce lipid deposits that have already formed, and reduce calcification, in addition to acting as a preservative and stabilizing agent The formulation also includes an effective amount of a permeation enhancer that facilitates penetration of the formulation components through cell membranes, tissues, and extracellular matrices, including the cornea. The "effective amount" of the permeation enhancer represents a concentration that is sufficient to provide a measurable increase in penetration of one or more of the formulation components through membranes, tissues, and extracellular matrices as just described. Suitable permeation enhancers include, by way of example, methylsulfonylmethane (MSM; also referred to as methyl sulfone), combinations of MSM with dimethylsulfoxide (DMSO), or a combination of MSM and, in a less preferred embodiment, DMSO, with MSM particularly preferred.

MSM is an odorless, highly water-soluble (34% w/v @ 79 °F) white crystalline compound with a melting point of 108-110 °C and a molecular weight of 94.1 g/mol. MSM serves as a multifunctional agent herein, insofar as the agent not only increases cell membrane permeability, but also acts as a "transport facilitating agent" (TFA) that aids in the transport of one or more formulation components to both the anterior and posterior of the eye. Furthermore, MSM *per se* provides medicative effects, and can serve as an anti-inflammatory agent as well as an analgesic. MSM also acts to improve oxidative metabolism in biological tissues, and is a source of organic sulfur, which assists in the reduction of scarring. MSM additionally possesses unique and beneficial solubilization properties, in that it is soluble in water, as noted above, but exhibits both hydrophilic and hydrophobic properties because of the presence of polar S=O groups and nonpolar methyl groups. The molecular structure of MSM also allows for hydrogen bonding with other molecules, i.e., between the oxygen atom of each S=O group and hydrogen atoms of other molecules, and for formation of van der Waal associations, i.e., between the methyl groups and nonpolar (e.g., hydrocarbyl) segments of other molecules. Ideally, the concentration of MSM in the present formulations is in the range of about 1.0 wt.% to 33 wt.%, preferably about 1.5 wt.% to 8.0 wt.%.

In this embodiment, the formulation also includes an agent that reduces the presence of AGEs, which are formed by reaction of glucose and other reducing sugars with proteins, lipoproteins, and DNA by a nonenzymatic "glycation" reaction. As described in U.S. Patent No. 6,337,350 to Rahbar et al., the reaction is initiated with the reversible formation of a Schiffs base by the coupling of a carbonyl group on a sugar molecule to an amino group on a second molecule (e.g., an amino terminus of a peptide or protein, or a free amino group on an amino acid side chain), followed by rearrangement to form a stable Amadori product. As explained in the aforementioned patent, both the Schiffs base and Amadori product further undergo a series of reactions, over time, in which crosslinking occurs, ultimately forming AGEs. AGEs, which are crosslinked macromolecules, generally crosslinked proteins and lipoproteins, stiffen connective tissue and lead to tissue damage. AGEs that have been identified to date include carboxymethyllysine, carboxyethyllysine, carboxymethylarginine, pentosidine, pyralline, pyrrolopyrridinium, arginine-lysine dimer, arginine pyridinium, cypentodine, piperidinedione enol, and vesperlysine. See Baynes et al. (1999) Diabetes 48:1-9.

The anti-AGE agent may be an AGE breaker, which acts to cleave glycated bonds and thus facilitate dissociation of already-formed AGEs. Suitable AGE breakers include, without limitation, L-carnosine, 3-phenacyl-4,5-dimethylthiazolium chloride (PTC), N-phenacylthiazolium bromide (PTB), and 3-phenacyl-4,5-dimethylthiazolium bromide (ALT-711, Alteon). The anti-AGE agent may also be selected from glycation inhibitors and AGE formation inhibitors. Representative such agents include aminoguanidine, 4-(2,4,6-trichlorophenylureido)phenoxyisobutyric acid, 4-[(3,4-dichlorophenylmethyl)2-chloro-phenylureido]phenoxyisobutyric acid, N,N'-bis(2-chloro-4-carboxyphenyl)formamidine, and combinations thereof.

The particularly preferred anti-AGE agent herein is L-carnosine, a natural histidine-containing dipeptide. L-carnosine is also a naturally occurring anti-oxidant, and thus provides multiple functions herein. By itself, L-carnosine does not penetrate through eye tissues, and this limitation has thus far limited its utility in ophthalmic compositions. In the present formulation, however, L-carnosine does penetrate sufficiently to exert a beneficial effect. In a preferred embodiment, L-carnosine represents approximately 0.2 wt.% to 5.0 wt.% of the formulation.

Optionally, the formulation also includes a microcirculatory enhancer, i.e., an agent that serves to enhance blood flow within the capillaries. The microcirculatory enhancer is preferably a phosphodiesterase (PDE) inhibitor, and most preferably an inhibitor of Type (I) PDE inhibitors. Such compounds, as will be appreciated by those of ordinary skill in the art, act to elevate intracellular levels of cyclic AMP (cAMP). A preferred microcirculatory enhancer is vinpocetine, also referred to as ethyl apovincamin-22-oate. Vinpocetine, a synthetic derivative of vincamine, a Vinca alkaloid, is particularly preferred herein because of its antioxidant properties and protection against excess calcium accumulation in cells. Vincamine is also useful as a microcirculatory enhancer herein, as are Vinca alkaloids other than vinpocetine. Preferably, the microcirculatory enhancer, e.g., vinpocetine, is present in an amount of about 0.01 wt.% to about 0.2 wt.%, preferably in the range of about 0.02 wt.% to about 0.1 wt.% of the formulation.

Other optional additives in the present formulations include secondary enhancers, i.e., one or more additional permeation enhancers. For example, formulation of the invention can contain added DMSO. Since MSM is a metabolite of DMSO (i.e., DMSO is enzymatically converted to MSM), incorporating DMSO into an MSM-containing formulation of the invention will tend to gradually increase the fraction of MSM in the formulation. DMSO also serves as a free radical scavenger, thereby reducing the potential for oxidative damage. If DMSO is added as a secondary enhancer, the amount is preferably in the range of about 1.0 wt.% to 2.0 wt.% of the formulation, and the weight ratio of MSM to DMSO is typically in the range of about 1:1 to about 50:1.

Other possible additives for incorporation into the formulations that are at least partially aqueous include, without limitation, thickeners, isotonic agents, buffering agents, and preservatives, providing that any such excipients do not interact in an adverse manner with any of the formulation's other components. It should also be noted that preservatives are not generally necessarily in light of the fact that the selected chelating agent and preferred AGE breakers themselves serve as preservatives. Suitable thickeners will be known to those of ordinary skill in the art of ophthalmic formulation, and include, by way of example, cellulosic polymers such as methylcellulose (MC), hydroxyethylcellulose (HEC), hydroxypropylcellulose (HPC), hydroxypropyl-methylcellulose (HPMC), and sodium carboxymethylcellulose (NaCMC), and other swellable hydrophilic polymers such as polyvinyl alcohol (PVA), hyaluronic acid or a salt thereof (e.g., sodium hyaluronate), and crosslinked acrylic acid polymers commonly referred to as "carbomers" (and available from B.F. Goodrich as Carbopol^{®} polymers). The preferred amount of any thickener is such that a viscosity in the range of about 15 cps to 25 cps is provided, as a solution having a viscosity in the aforementioned range is generally considered optimal for both comfort and retention of the formulation in the eye. Any suitable isotonic agents and buffering agents commonly used in ophthalmic formulations may be used, providing that the osmotic pressure of the solution does not deviate from that of lachrymal fluid by more than 2-3% and that the pH of the formulation is maintained in the range of about 6.5 to about 8.0, preferably in the range of about 6.8 to about 7.8, and optimally at a pH of about 7.4. Preferred buffering agents include carbonates such as sodium and potassium bicarbonate.

The formulations of the invention also include a pharmaceutically acceptable ophthalmic carrier, which will depend on the particular type of formulation. For example, the formulations of the invention can be provided as an ophthalmic solution or suspension, in which case the carrier is at least partially aqueous. The formulations may also be ointments, in which case the pharmaceutically acceptable carrier is composed of an ointment base. Preferred ointment bases herein have a melting or softening point close to body temperature, and any ointment bases commonly used in ophthalmic preparations may be advantageously employed. Common ointment bases include petrolatum and mixtures of petrolatum and mineral oil.

The formulations of the invention may also be prepared as a hydrogel, dispersion, or colloidal suspension. Hydrogels are formed by incorporation of a swellable, gel-forming polymer such as those set forth above as suitable thickening agents (i.e., MC, HEC, HPC, HPMC, NaCMC, PVA, or hyaluronic acid or a salt thereof, e.g., sodium hyaluronate), except that a formulation referred to in the art as a "hydrogel" typically has a higher viscosity than a formulation referred to as a "thickened" solution or suspension. In contrast to such preformed hydrogels, a formulation may also be prepared so as to form a hydrogel *in situ* following application to the eye. Such gels are liquid at room temperature but gel at higher temperatures (and thus termed "thermoreversible" hydrogels), such as when placed in contact with body fluids. Biocompatible polymers that impart this property include acrylic acid polymers and copolymers, N-isopropylacrylamide derivatives, and ABA block copolymers of ethylene oxide and propylene oxide (conventionally referred to as "poloxamers" and available under the Pluronic^{®} tradename from BASF-Wyandotte). The formulations can also be prepared in the form of a dispersion or colloidal suspension. Preferred dispersions are liposomal, in which case the formulation is enclosed within "liposomes," microscopic vesicles composed of alternating aqueous compartments and lipid bilayers. Colloidal suspensions are generally formed from microparticles, i.e., from microspheres, nanospheres, microcapsules, or nanocapsules, wherein microspheres and nanospheres are generally monolithic particles of a polymer matrix in which the formulation is trapped, adsorbed, or otherwise contained, while with microcapsules and nanocapsules, the formulation is actually encapsulated. The upper limit for the size for these microparticles is about 5µ to about 10µ.

The formulations may also be incorporated into a sterile ocular insert that provides for controlled release of the formulation over an extended time period, generally in the range of about 12 hours to 60 days, and possibly up to 12 months or more, following implantation of the insert into the conjunctiva, sclera, or pars plana, or into the anterior segment or posterior segment of the eye. One type of ocular insert is an implant in the form of a monolithic polymer matrix that gradually releases the formulation to the eye through diffusion and/or matrix degradation. With such an insert, it is preferred that the polymer be completely soluble and or biodegradable (i.e., physically or enzymatically eroded in the eye) so that removal of the insert is unnecessary. These types of inserts are well known in the art, and are typically composed of a water-swellable, gel-forming polymer such as collagen, polyvinyl alcohol, or a cellulosic polymer. Another type of insert that can be used to deliver the present formulation is a diffusional implant in which the formulation is contained in a central reservoir enclosed within a permeable polymer membrane that allows for gradual diffusion of the formulation out of the implant. Osmotic inserts may also be used, i.e., implants in which the formulation is released as a result of an increase in osmotic pressure within the implant following application to the eye and subsequent absorption of lachrymal, fluid.

In another embodiment of the invention, a sterile ophthalmic formulation is provided that contains: a biocompatible chelating agent at a concentration of at least 0.6% by weight; an effective permeation-enhancing amount of methylsulfonylmethane, preferably although not necessarily representing about 1.0 wt.% to about 33 wt.% of the formulation, more preferably about 1.5 wt.% to about 8.0 wt.% of the formulation; and a pharmaceutically acceptable ophthalmic carrier. Suitable biocompatible chelating agents, carriers, optional additives, and delivery systems are as described above. In this embodiment, it is preferred that the carrier be distilled or deionized water. An exemplary biocompatible chelating agent is EDTA or an ophthalmologically acceptable salt thereof, and is present at a concentration no higher than 10 wt.% of the formulation. The formulation can also contain about 0.5 wt.% to about 30 wt.% L-carnosine, about 0.1 wt.% to about 0.5 wt.% 3-phenacyl-4,5-dimethylthiazolium chloride, about 1.0 wt.% to about 2.0 wt.% dimethyl sulfoxide, about 0.01 wt.% to about 0.2 wt.%, preferably about 0.02 wt.% to about 0.1 wt.% vinpocetine, and a buffering agent or system effective to provide the formulation with a pH in the range of about 6.5 to about 8.0, preferably about 6.8 to about 7.8, and ideally about 7.4.

In a further embodiment of the invention, a sterile ophthalmic formulation is provided that contains: a biocompatible chelating agent at a concentration of at least 0.6% by weight; an effective AGE-reducing concentration of L-carnosine, generally although not necessarily representing about 0.5 wt.% to 30 wt.% of the formulation; and a pharmaceutically acceptable ophthalmic carrier. Suitable biocompatible chelating agents, carriers, optional additives, and delivery systems are as described earlier herein, and it is preferred that the carrier be distilled or deionized water. Preferably, the biocompatible chelating agent is EDTA or an ophthalmologically acceptable salt thereof, present at a concentration no higher than 10 wt.% of the formulation. The formulation can also contain about 0.01 wt.% to about 0.2 wt.%, preferably about 0.02 wt.% to about 0.1 wt.% vinpocetine, and a buffering agent or system which, as above, is effective to provide the formulation with a pH in the range of about 6.5 to about 8.0, preferably about 6.8 to about 7.8, and ideally about 7.4.

The formulations of the invention are useful in treating a wide variety of adverse ocular conditions, including conditions, diseases or disorders of the cornea, retina, lens, sclera, and anterior and posterior segments of the eye. The formulations are particularly useful in treating adverse ocular conditions associated with the aging process and/or oxidative and free radical damage to the eye. By way of example and not limitation, the formulations are useful in treating the following adverse ocular conditions that are generally associated with aging: hardening, opacification, reduction of pliability, and yellowing of the lens; yellowing and opacification of the cornea; presbyopia; clogging of the trabeculum, leading to intraocular pressure build-up and glaucoma; increased floaters in the vitreous humor; stiffening and reduction of the dilation range of the iris; age-related macular degeneration; formation of atherosclerotic and other lipid deposits in retinal arteries; dry eye syndrome; development of cataracts, including secondary cataracts; photophobia, problems with glare and a decrease in the sensitivity and light level adaptation ability of the rods and cones of the retina; arcus senilis; narrowing of the pupil; loss in visual acuity, including decreased contrast sensitivity, color perception, and depth perception; loss of night vision; decreased lens accommodation; macular edema; macular scarring; and band keratopathy. The aging individual generally suffers from more than one of these conditions, normally necessitating the self-administration of two or more different pharmaceutical products. As the formulation of the invention is useful for treating all of these conditions, no additional products are needed, and, therefore, the inconvenience and inherent risk of using multiple pharmaceutical products are eliminated. Additional adverse ocular conditions that can be treated using the present formulations include keratoconus and ocular surface growths such as pingueculae and pterygia. It should also be emphasized that the formulations can be used to improve the visual acuity, including contrast sensitivity, color perception, and depth perception, in any mammalian individual whether or not the individual is afflicted with an adverse visual condition.

The invention also pertains to ocular inserts for the controlled release of a biocompatible chelating agent as described above and/or an anti-AGE agent, without an enhancer. These ocular inserts may be implanted into any region of the eye, including the sclera and the anterior and posterior segments. One such insert is composed of a controlled release implant containing a formulation that consists essentially of the biocompatible chelating agent, preferably EDTA or an ophthalmologically acceptable salt thereof, and a pharmaceutically acceptable carrier. Another such insert is composed of a controlled release implant containing a formulation that consists essentially of the anti-AGE agent, preferably L-carnosine, and a pharmaceutically acceptable carrier. The insert may be a gradually but completely soluble implant, such as may be made by incorporating swellable, hydrogel-forming polymers into an aqueous liquid formulation as described elsewhere herein. The insert may also be insoluble, in which case the agent is released from an internal reservoir through an outer membrane via diffusion or osmosis as also described elsewhere herein.

It is to be understood that while the invention has been described in conjunction with the preferred specific embodiments thereof, the description above as well as the examples that follow are intended to illustrate and not limit the scope of the invention. Other aspects, advantages, and modifications within the scope of the invention will be apparent to those skilled in the art to which the invention pertains.

### EXAMPLE 1

An eye drop formulation of the invention, Formulation 1, was prepared as follows: High purity de-ionized (DI) water (500 ml) was filtered via a 0.2 micrometer filter. MSM (27 g), EDTA (13 g), and L-carnosine (5 g) were added to the filtered DI water, and mixed until visual transparency was achieved, indicating dissolution. The mixture was poured into 10 mL bottles each having a dropper cap. On a weight percent basis, the eye drops had the following composition:

| | |
|---|---|
| Purified de-ionized water | 91.74 wt.% |
| MSM | 4.95 wt.% |
| Di-sodium EDTA | 2.39 wt.% |
| L-Carnosine | 0.92 wt.%. |

### EXAMPLE 2

Formulation 1 was evaluated for efficacy in treating four subjects, all males between 52 and 84 years of age of mixed ethnicity. Subject 1 was in his fifties and had no visual problems or detectable abnormalities of the eye. Subjects 2 and 3 were in their fifties and had prominent arcus senilis around the cornea periphery in both eyes but no other adverse ocular conditions (arcus senilis is typically considered to be a cosmetic blemish). Subject 4 was in his eighties and was suffering from cataracts and Salzmann's nodules, and reported extreme photophobia and problems with glare. This subject was having great difficulty reading newspapers, books, and information on a computer screen, because of the glare and loss in visual clarity.

The formulation was administered to the subjects, one drop (approximately 0.04 mL) to each eye, two to four times per day for a period of over 12 months. All subjects were examined by an ophthalmologist during and after 12 months. No side effects, other than minor temporary irritation at the time of administering the formulation in the eye, were reported or observed by the subjects or the ophthalmologist. All four subjects completed the study.

All subjects noticed subjective changes 4 weeks into the study. At this stage, the changes reported by the subjects included increased brightness, improved clarity of vision, and reduced glare (particularly Subject 4).

After 8 weeks, the following changes were noted: All four subjects reported greatly improved vision with regard to clarity and contrast, and indicated that daytime colors appeared to increase in brilliance. Subject 1's eyesight improved from 20/25 (after correction) to better than 20/20 (with the same correction), and his eyes turned a deeper shade of blue. Subjects 2 and 3 exhibited a significant reduction of the arcus senilis.

For Subject 4, whose vision originally with best correction had been 20/400 in his left eye and 20/200 in his right eye and had acute photophobia and glare. The glare and photophobia were reduced, and the subject started to read books, newspapers, and information on the computer screen again. The visual acuity in his right eye improved significantly, from 20/200 (with correction) to 20/60 (pinhole) (with the same correction). In his left eye, his visual acuity improved as well, from 20/400 to 20/200 (with the same correction). In his left eye, he continued to have a central dark spot due to macular scarring.

After 16 weeks, the following changes were noted: All subjects reported continuing improvement of vision, including night vision, as well as improved contrast sensitivity and continued improvement in color perception. Subject 1's eyesight continued to improve, from 20/20 (after correction) to 20/15 (with the same correction). Subjects 2 and 3 continued to exhibit a reduction of the arcus senilis.

Subject 4 reported a further reduction in glare and photophobia, and further improvements in the ease of reading books, newspapers, and information on the computer screen. Subject 4 also reported that nighttime glare had been eliminated. The subject was now comfortable in daylight without need for dark glasses, and without suffering severe problems with glare. The visual acuity in his right eye improved from 20/60 (pinhole) to 20/50 (pinhole) In his left eye his visual acuity also improved, from 20/200 to 20/160 (with same correction). In his left eye, he continued to have a central dark spot due to macular scarring.

After eight months, Subject 4's vision in his right eye improved from 20/50 (pinhole) to 20/40 (pinhole) In his left eye his visual acuity improved from 20/160 to 20/100 (with same correction). The dark spot in the left eye started dissipating, and he could read hazily through the formerly dark spot. At this time his contrast sensitivity was also measured. His cataracts were measured at a 4+ (on a scale of 0-4, 4 being the highest). The central macular scar was barely visible to the ophthalmologist due to haziness of the optical path.

After 10 months, Subject 1's visual acuity further improved from 20/15 to 20/10 (with the same correction).

After further 2 months; i.e., after a total of 12 months, Subject 4's vision continued to improve. The subject could now read books, newspapers, and the computer screen without any problems. The subject also showed improvement in cataracts (went from 4+ to 3-4+ on a 0-4 scale). The optical path clarity had improved enough that the macular scar was clearly visible to the ophthalmologist. In contrast sensitivity there was a 40% to 100% improvement. In Snellen acuity, he went from from 20/40 to 20/30 (pinhole) in his right eye, and from 20/100 to 20/80 in his left eye.

### EXAMPLE 3

A second eye drop formulation of the invention, Formulation 2, was prepared as follows:

High purity de-ionized (DI) water (500 ml) was filtered via a 0.2 micrometer filter. MSM (13.5 g), EDTA (6.5 g), and L-carnosine (5.0 g) were added to the filtered DI water, and mixed until visual transparency was achieved, indicating dissolution. The mixture was poured into 10 mL bottles each having a dropper cap. On a weight percent basis, the eye drop composition had the following components:

| | |
|---|---|
| Purified de-ionized water | 95.24 wt.% |
| MSM | 2.57 wt.% |
| Di-sodium EDTA | 1.24 wt.% |
| L-Carnosine | 0.95 wt.% |

### EXAMPLE 4

Subsequent to the experimentation described in Example 2, a detailed and controlled follow-on study was carried out using a slightly weaker eye drop formulation, Formulation 2 (prepared as described in Example 3). Placebo eye drops were also prepared and administered. The placebo drops were composed of a commercially obtained sterile saline solution in the form of a buffered isotonic aqueous solution (containing boric acid, sodium borate, and sodium chloride with 0.1 wt.% sorbic acid and 0.025 wt.% di-sodium EDTA as preservatives).

The study was double-masked, in that except for one positive control, neither the patient nor the ophthalmologist knew whether they were given the formulation eye drops or a saline solution. The patients were randomized to receive either the study formulation or saline solution.

The study involved five subjects, of which 3 subjects were given the eye drops of Formulation 2 and 1 subject was given placebo eye drops. In addition, 1 subject was given the higher-strength eye drops of Formulation 1. One drop (approximately 0.04 mL) was administered to each eye, two to four times daily for a period of 8 weeks. The drops were administered to both eyes of each subject. The study participants were multiethnic and 20% female, 80% male.

The baseline and follow-on testing by the ophthalmologist included: automated refraction; corneal topography; external photographs; wavefront photographs; visual acuity with spectacle correction at distance and at 14 inches; contrast sensitivity testing using the Vision Sciences Research Corporation (San Ramon, California) Functional Acuity Contrast Test (FACT) chart; pupil examination and pupil size measurement; slit lamp examination; intraocular pressure measurement; and dilated fundus examination.

After 8 weeks, the subjects were examined again. The contrast sensitivity results for each subject are shown in Table 1, and all the results are summarized in Table 2.

| Table 1 | | | | | | | | | | | |
|---|---|---|---|---|---|---|---|---|---|---|---|
| Subject | | 1 | | 2 | | 3 | | 4⁵ | | 5⁶ | |
| Right (R) or Left (L) Eye | | R | L | R | L | R | L | R | L | R | L |
| Contrast Sensitivity (CS)' | | | | | | | | | | | |
| 1.5 cpd² | log₁₀ CS before | 1.85 | 1.56 | 1.70 | 1.70 | 2.00 | 1.85 | 1.56 | 1.70 | 1.85 | 1.70 |
| | log₁₀ CS after | 2.00 | 1.85 | 1.85 | 1.85 | 2.00 | 1.85 | 1.85 | 1.85 | 1.85 | 1.56 |
| | log₁₀ unit change³ | 0.15 | 0.29 | 0.15 | 0.15 | 0.00 | 0.00 | 0.29 | 0.15 | 0.00 | -.14 |
| | percent improved⁴ | 8 | 19 | 9 | 9 | 0 | 0 | 19 | 9 | 0 | -8 |
| | | | | | | | | | | | |
| 3 cpd | log₁₀ CS before | 1.90 | 1.76 | 1.90 | 1.90 | 1.90 | 1.90 | 1.76 | 1.90 | 1.76 | 1.90 |
| | log₁₀ CS after | 2.06 | 1.90 | 1.90 | 2.06 | 2.06 | 2.06 | 2.20 | 2.06 | 1.90 | 1.76 |
| | log₁₀ unit change | 0.16 | 0.14 | 0.00 | 0.16 | 0.16 | 0.16 | 0.44 | 0.16 | 0.14 | -.14 |
| | percent improved | 8 | 8 | 0 | 8 | 8 | 8 | 26 | 8 | 8 | -8 |
| | | | | | | | | | | | |
| 6 cpd | log₁₀ CS before | 1.81 | 1.81 | 1.95 | 2.11 | 1.95 | 1.95 | 1.65 | 1.81 | 1.81 | 1.81 |
| | log₁₀ CS after | 1.95 | 1.81 | 2.11 | 1.95 | 2.11 | 2.11 | 2.11 | 2.11 | 1.81 | 1.95 |
| | log₁₀ unit change | 0.14 | 0.00 | 0.16 | -.16 | 0.16 | 0.16 | 0.46 | 0.30 | 0.00 | 0.14 |
| | percent improved | 8 | 0 | 8 | -7 | 8 | 8 | 27 | 17 | 0 | 8 |
| | | | | | | | | | | | |
| 12 cpd | log₁₀ CS before | 1.34 | 1.18 | 1.78 | 1.78 | 1.78 | 1.78 | 1.18 | 1.48 | 1.48 | 1.63 |
| | log₁₀ CS after | 1.63 | 1.48 | 1.78 | 1.78 | 1.78 | 1.78 | 1.93 | 1.78 | 1.63 | 1.48 |
| | log₁₀ unit change | 0.29 | 0.30 | 0.00 | 0.00 | 0.00 | 0.00 | 0.75 | 0.30 | 0.15 | -.15 |
| | percent improved | 22 | 26 | 0 | 0 | 0 | 0 | 64 | 20 | 11 | -10 |
| | | | | | | | | | | | |
| 18 cpd | log₁₀ CS before | 0.90 | 1.08 | 1.23 | 1.23 | 1.23 | 1.30 | 0.60 | 1.23 | 0.90 | 1.23 |
| | log₁₀ CS after | 1.36 | 1.36 | 1.36 | 1.36 | 1.52 | 1.52 | 1.66 | 1.52 | 1.36 | 1.36 |
| | log₁₀ unit change | 0.46 | 0.28 | 0.13 | 0.13 | 0.29 | 0.22 | 1.06 | 0.29 | 0.46 | 0.13 |
| | percent improved | 51 | 27 | 11 | 11 | 23 | 12 | 176 | 23 | 51 | 11 |

| | | | | | | | | | | | |
|---|---|---|---|---|---|---|---|---|---|---|---|
| ¹Contrast sensitivity (CS) is the reciprocal of the contrast at threshold, i.e., one divided by the lowest contrast at which forms or lines can be recognized. Log of the contrast sensitivity values is a generally accepted method for comparing contrast sensitivities. ²cpd = cycles per degree for the spatial frequency ³Log unit change = log₁₀(CS after treatment) - log₁₀(CS before treatment) ⁴Percent improved = [log₁₀(CS after treatment)/log₁₀(CS before treatment)-1] x 100 ⁵Positive control ⁶placebo | | | | | | | | | | | |

| Table 2 | | | | |
|---|---|---|---|---|
| | | Formulation 1 (positive control) n=1 | Formulation 2 (study subjects) n=3 | Saline Solution (placebo) n=1 |
| Pupil Dilation | | +20% | +8% | 0% |
| Snellen Acuity (distance vision) | | +17.5% | +7.5% | -15% |
| Snellen Acuity (near vision) | | 0% | +10% | 0% |
| Auto refraction | | +8% | +8% | 0% |

| Contrast Sensitivity¹ | | | | |
|---|---|---|---|---|
| 1.5 cpd² | percent improved³ | 14% | 7.5% | -4% |
| | log unit change⁴ | 0.22 | 0.12 | -0.08 |
| | | | | |
| 3 cpd | percent improved | 17% | 6.8% | 0% |
| | log unit change | 0.33 | 0.12 | 0 |
| | | | | |
| 6 cpd | percent improved | 22% | 4% | 4% |
| | log unit change | 0.38 | 0.08 | 0.08 |
| | | | | |
| 12 cpd | percent improved | 42% | 7.9% | 0% |
| | log unit change | 0.53 | 0.10 | 0 |
| | | | | |
| 18 cpd | percent improved | 99.5% | 22.2% | 31.0% |
| | log unit change | 0.68 | 0.24 | 0.26 |
| Wavefront (image tightness) | | +23% | +38% | 0% |

| | | | | |
|---|---|---|---|---|
| ¹Contrast sensitivity (CS) is the reciprocal of the contrast at threshold, i.e., one divided by the lowest contrast at which forms or lines can be recognized. Log of the contrast sensitivity values is a generally accepted method for comparing contrast sensitivities. ²cpd = cycles per degree for the spatial frequency ³Percent improved = [log₁₀(CS aftertreatment)/log₁₀(CS before treatment)-1] x 100 ⁴Log unit change = log₁₀(CS after treatment) - log₁₀(CS before treatment) | | | | |

Subjects treated with Formulation 1 and Formulation 2 all showed very significant improvements, including improved smoothness and regularity of the cornea, improved accommodative/focusing ability, more uniform and stable tear film, and decreased yellowing of the cornea and lens. Subjects to whom the placebo was given did not exhibit any significant change. All subjects reported improved ability to see road signs at a distance, brighter and more vivid colors, and improved night vision.

### EXAMPLE 5

Formulation 1 was evaluated for efficacy in a 46-year-old male subject. Prior to treatment, the subject had no severe visual problems or eye abnormalities, but he did require bifocals to correct refractive errors in both eyes.

The subject was examined by an independent ophthalmologist prior to treatment and again following eight weeks of treatment. Tests performed included: Snellen visual acuity examinations for distance (20 feet) and near (14 inches) vision, autorefraction, pupil dilation (pupillometer maximum scotopic pupil size), slit lamp examination, automated corneal topography mapping, contrast sensitivity (functional acuity contrast test), automated wavefront aberration mapping, and photographs of the anterior segment.

Treatment consisted of the topical instillation of one drop (approximately 0.04 mL) of Formulation 1 in each eye two to four times per day for eight weeks. Results of this treatment were as follows:

No irritation, redness, pain, or other adverse effects were observed by the ophthalmologist or reported by the subject, other than transient minor eye irritation at the time of eye drop administration.

Snellen visual acuity: Using the same refractive correction, distance visual acuity improved from 20/25+1 to 20/20 in the right eye, and from 20/20-2 to 20/20 in the left eye. Near vision was unchanged at 20/50 in both eyes.

Autorefraction: The right eye was unchanged: spherical -3.75; astigmatism +2.5 at axis of 24 degrees. The left eye showed slight improvement: spherical decreased from -4.00 to -3.75; astigmatism decreased from +3.50 at 175 degrees to +3.25 at 179 degrees.

Pupil dilation: Both eyes improved from 5.0 to 6.0 mm.

Slit lamp examination: The retinas appeared unchanged, and no cataracts were observed during either examination.

Corneal topography: Improved smoothness and regularity of the cornea were observed in both eyes. The ophthalmologist remarked that the improvement may have been due to a more uniform and stable tear film.

Contrast sensitivity: Measurements are shown in Table 3.

| Table 3 | | | | | | | | | | |
|---|---|---|---|---|---|---|---|---|---|---|
| CPD* | 1.5 | | 3 | | 6 | | 12 | | 18 | |
| Eye | R | L | R | L | R | L | R | L | R | L |
| Before | 6 | 7 | 6 | 7 | 5 | 6 | 3 | 5 | 1 | 5 |
| After | 8 | 8 | 9 | 8 | 8 | 8 | 8 | 7 | 8 | 7 |

| | | | | | | | | | | |
|---|---|---|---|---|---|---|---|---|---|---|
| *CPD = cycles per degree (spatial frequency of pattern) | | | | | | | | | | |

These data indicate a consistent, significant improvement in contrast sensitivity.

Automated wavefront mapping: For the right eye, spherical aberration was essentially unchanged (+0.15660 to +0.15995). Retinal image formation improved from 60x70 to 45x70 minutes of arc, which represents a 25% tighter image formation. For the left eye: Spherical aberration decreased from +0.14512 to +0.09509, representing a 34.4% improvement. Retinal image formation improved with an estimated 20% tighter image.

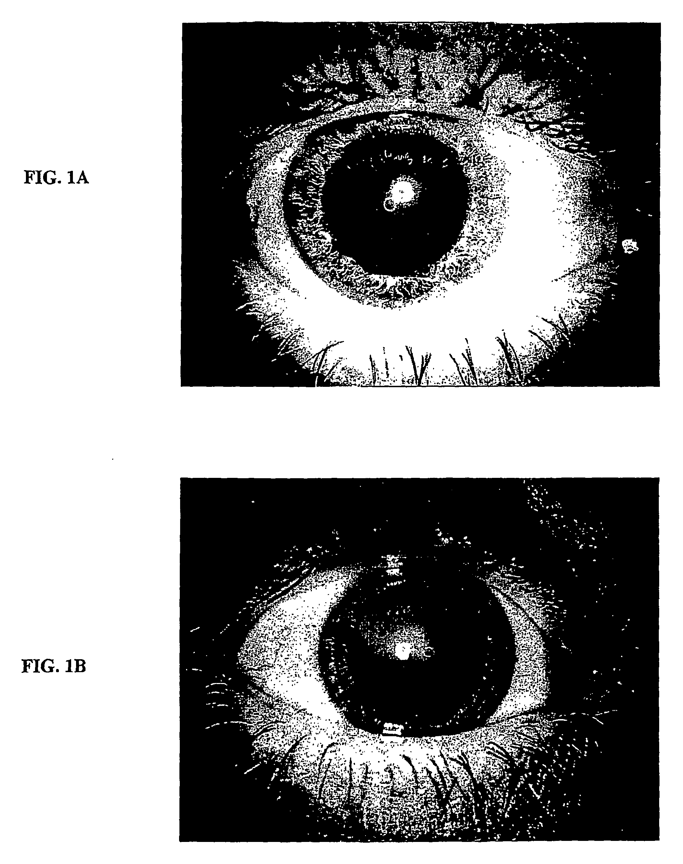

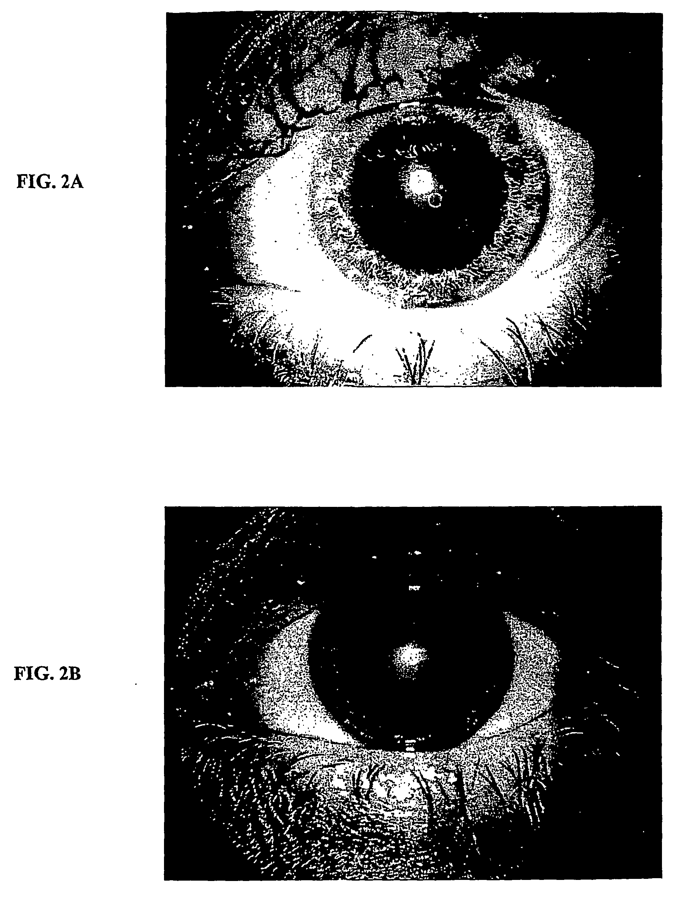

Photographs of anterior segment, FIG. 1A (OD, before treatment), FIG. 1B (OD, after treatment), FIG. 2A (OS, before treatment), and FIG. 2B (OS, after treatment): Iris color changed to a darker blue; the degree of change was reported as "striking." The change was likely due to a decrease in the yellowing of the cornea.

In addition, the subject reported that, following treatment, he switched to lower power prescription glasses and no longer required bifocals. He made the following remarks: "I have been using the eye drops for about eight weeks, and my eyesight has significantly improved. I can see colors more vividly. I have replaced my bifocals with my older, lower power non-bifocals. I can see much better in the distance and do not need reading glasses. My eyes have become a darker blue like my original eye color, and my night vision has improved."

### EXAMPLE 6

Formulation 1 was evaluated for efficacy in a 60-year-old male subject. Prior to treatment, the subject had no serious visual problems or eye abnormalities other than refractive errors in both eyes.

The subject was examined by an independent ophthalmologist prior to treatment and again following seven weeks of treatment. Tests performed included: Snellen visual acuity examinations for distance (20 feet) and near (14 inches) vision, autorefraction, pupil dilation (pupillometer maximum scotopic pupil size), slit lamp examination, automated corneal topography mapping, contrast sensitivity (functional acuity contrast test), automated wavefront aberration mapping, and photographs of the anterior segment.

Treatment consisted of the topical instillation of one drop (approximately 0.04 mL) of Formulation 1 in each eye two to four times per day for seven weeks. Results of this treatment were as follows:

No irritation, redness, pain, or other adverse effects were observed by the ophthalmologist or reported by the subject, other than transient minor eye irritation at the time of eye drop administration.

Snellen visual acuity: Using the same refractive correction (intentionally undercorrected in the left eye), distance visual acuity remained unchanged at 20/15 in the right eye, and improved from 20/40-2 to 20/40 in the left eye. Near vision declined from 20/70 to 20/100 in the right eye (likely due to overcorrection for distance), and improved from 20/40-2 to 20/25 in the left eye.

Autorefraction: The right eye had an unchanged spherical measurement (-6.00) and a slight improvement in astigmatism (+0.75 at 115 degrees to +0.50 at 113 degrees). The left eye showed slight improvement: spherical went from -8.25 to -8.00; astigmatism was unchanged, from +1.00 at 84 degrees to +1.00 at 82 degrees.

Pupil dilation: The right eye improved from 4.0 to 4.5 mm, and the left eye was unchanged at 4.0 mm.

Slit lamp examination: The retinas appeared unchanged, and minimal cataracts were observed during both examinations.

Corneal topography: Improved smoothness and regularity of the cornea were observed in both eyes. The ophthalmologist remarked that the improvement may have been due to a more uniform and stable tear film.

Contrast sensitivity: Measurements are shown in Table 4.

| Table 4 | | | | | | | | | | |
|---|---|---|---|---|---|---|---|---|---|---|
| CPD* | 1.5 | | 3 | | 6 | | 12 | | 18 | |
| Eye | R | L | R | L | R | L | R | L | R | L |
| Before | 8 | 6 | 7 | 6 | 6 | 6 | 4 | 3 | 3 | 4 |
| After | 9 | 8 | 8 | 7 | 7 | 6 | 6 | 5 | 6 | 6 |

| | | | | | | | | | | |
|---|---|---|---|---|---|---|---|---|---|---|
| *CPD = cycles per degree (spatial frequency of pattern) | | | | | | | | | | |

These data indicate a consistent, significant improvement in contrast sensitivity.

Automated wavefront mapping: For the right eye: Spherical aberration decreased from +0.01367 to +0.00425, a 69% improvement. Retinal image formation improved from 80x80 to 70x65 minutes of arc, which represents a 28.9% tighter image formation. For the left eye: Spherical aberration decreased from +0.04687 to -0.00494, representing a >100% improvement. Retinal image formation improved from 150x150 to 100x100 minutes of arc, which represents a 33% tighter image formation. The ophthalmologist remarked at the second examination: "Overall spherical aberration is closer to that of a young healthy eye rather than a 60-year-old eye."

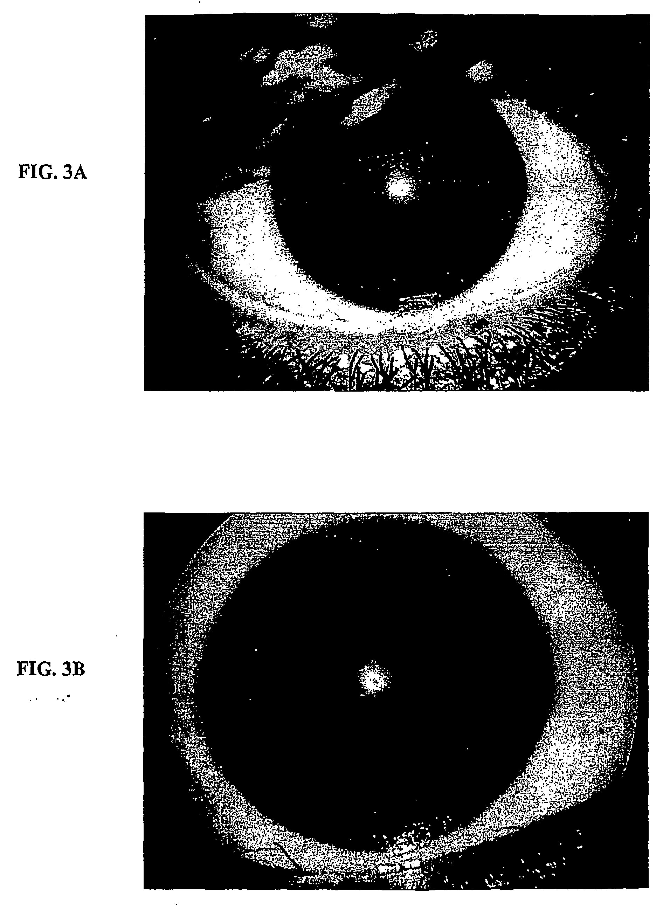

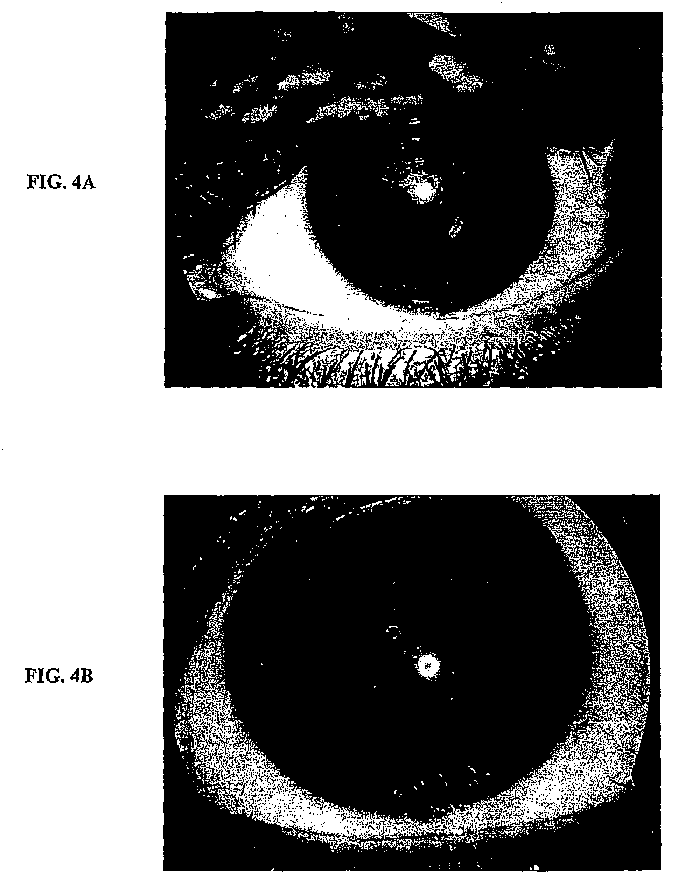

Photographs of anterior segment, FIG. 3A (OD, before treatment), FIG. 3B (OD, after treatment), FIG. 4A (OS, before treatment), and FIG. 4B (OS, after treatment): Observed were an apparent decrease in lens opacity, reduced yellowing of the crystalline lens, and improved corneal clarity.

In addition, the subject stated: "I have used these eye drops for about seven weeks. I can see a golf ball at 300 yards, whereas it was barely visible at 220 yards before. My vision vastly improved, especially in seeing road signs in the distance. I see colors much more brightly and vividly."

### EXAMPLE 7

The ocular pharmacokinetic behavior of EDTA, when administered as a component of Formulation 1, was evaluated in rabbits over a period of five days. Two healthy male rabbits, each approximately 2.5 to 3 Kg in body weight, were used for the study.

On day 1 of the study, one drop of Formulation 1 was topically instilled in each eye of both rabbits (four eyes total). No additional eye drops were administered during the course of the study. Samples of aqueous humor were extracted at 15 min, 30 min, 1 hr, 4 hrs, 3 days, and 5 days following administration (as indicated in the following table). Vitreous humor was extracted at 5 days following administration from all four eyes. The concentration of EDTA was measured in all the samples of aqueous humor and vitreous humor by HPLC analysis.

The results of the study are summarized in Table 5.

| Table 5 | | | | |
|---|---|---|---|---|
| | Concentration of EDTA (micrograms per milliliter) | | | |
| | Rabbit 101 | | Rabbit 102 | |
| Aqueous humor: | Right Eye | Left Eye | Right Eye | Left Eye |
| 15 min | 1.3 | | | |
| 30 min | | | 10.7 | |
| 1 hr | | 5.3 | | |
| 4 hrs | | | | 0.9 |
| 3 days | 0.5 | 0.4 | 0.5 | 0.7 |
| 5 days | 0.6 | 0.5 | 0.4 | 0.6 |

| Vitreous humor: | | | | |
|---|---|---|---|---|
| 5 days | 0.6 | 0.5 | 0.7 | 0.6 |

These results show that Formulation 1 delivers EDTA to the anterior chamber of the eye (aqueous humor) very rapidly: a concentration of 10.7 µg/mL is reached at only 30 minutes following administration. Because the aqueous humor is completely flushed from the anterior chamber approximately every 90 minutes, compounds from conventional eye drop formulations are typically not detected in the aqueous humor at four hours following administration. We, however, observed significant concentrations of EDTA in the aqueous humor even at five days following administration. Our data also show that EDTA reached the vitreous humor, where it was present in almost the same concentration as in the aqueous humor. It is thus likely that the vitreous humor (and probably adjacent tissues) was acting as a reservoir for the absorbed EDTA, with some of this EDTA diffusing back into the aqueous humor over time.

The demonstrated penetration of EDTA from Formulation 1 into the posterior segment of the eye, including the vitreous humor, indicates the potential of the inventive formulation to deliver therapeutic agents to the posterior of the eye when administered as eye drops. Such drug delivery to the posterior of the eye allows for the treatment of many eye conditions, diseases, and disorders, including age related macular degeneration, macular edema, glaucoma, cell transplant rejection, infections, and uveitis.

These examples indicate that topical drops composed of the multifunction agents MSM and EDTA, with the addition of the L-carnosine AGE breakers, significantly improved the quality of both day and night vision (visual acuity), greatly improved contrast sensitivity, improved pupil dilation, produced a more uniform and stable tear film, reduced arcus senilis, and greatly reduced glare and the discomfort associated with photophobia. No adverse pathological changes or reduction in acuity were observed.
Various preferred features and embodiments of the invention will now be described by way of example with reference to the following numbered paragraphs (paras.):
1. A sterile ophthalmic formulation, comprising:
   a biocompatible chelating agent at a concentration of at least 0.6% by weight;
   an effective permeation-enhancing concentration of a permeation enhancer;
   an anti-AGE agent selected from AGE breakers, AGE formation inhibitors, and glycation inhibitors; and
   a pharmaceutically acceptable ophthalmic carrier.
2. The formulation of para. 1, wherein the carrier is at least partially aqueous.
3. The formulation of para. 2, comprising a solution.
4. The formulation of para. 2, comprising a suspension.
5. The formulation of para. 2, wherein the carrier further includes a water-swellable polymer and the formulation comprises a hydrogel.
6. The formulation of para. 2, wherein the carrier comprises a thermoreversible hydrogel-forming polymer such that the formulation forms a hydrogel *in situ* following ocular administration.
7. The formulation of para. 1, wherein the carrier is an ointment base, and the formulation comprises an ointment.
8. A sterile ophthalmic delivery system comprising a liposomal dispersion of the formulation of para. 1.
9. The delivery system of para. 1, comprising a colloidal suspension of microspheres, nanospheres, microcapsules, or nanocapsules containing the formulation of para. 1.
10. The formulation of para. 1, wherein the biocompatible chelating agent is selected from ethylenediamine tetraacetic acid (EDTA), cyclohexanediamine tetraacetic acid (CDTA), hydroxyethylethylenediamine triacetic acid (HEDTA), diethylenetriamine pentaacetic acid (DTPA), dimercaptopropane sulfonic acid (DMPS), dimercaptosuccinic acid (DMSA), aminotrimethylene phosphonic acid (ATPA), citric acid, ophthalmologically acceptable salts thereof, and combinations of any of the foregoing.
11. The formulation of para. 10, wherein the biocompatible chelating agent is selected from EDTA and ophthalmologically acceptable salts thereof.
12. The formulation of para. 11, wherein the biocompatible chelating agent is EDTA.
13. The formulation of para. 11, wherein the biocompatible chelating agent is an ophthalmologically acceptable EDTA salt.
14. The formulation of para. 13, wherein the ophthalmologically acceptable EDTA salt is selected from diammonium EDTA, disodium EDTA, dipotassium EDTA, triammonium EDTA, trisodium EDTA, tripotassium EDTA, calcium disodium EDTA, and combinations thereof.
15. The formulation of para. 1, wherein the chelating agent is selected from chelating antibiotics, chelating agents containing two or more chelating nitrogen atoms, phosphates, and deferoxamine.
16. The formulation of para. 15, wherein the chelating agent is a chelating antibiotic selected from chloroquine and tetracycline.
17. The formulation of para. 15, wherein the chelating agent is selected from pyrophosphates, tripolyphosphates, hexametaphosphates, and combinations thereof.
18. The formulation of para. 1, wherein the permeation enhancer is selected from methylsulfonylmethane, dimethyl sulfoxide, and combinations thereof.
19. The formulation of para. 20, wherein the permeation enhancer is methylsulfonylmethane.
20. The formulation of para. 18, comprising methylsulfonylmethane and dimethyl sulfoxide at a weight ratio of approximately 1:1 to about 50:1.
21. The formulation of para. 1, wherein the anti-AGE agent is an AGE breaker.
22. The formulation of para. 17, wherein the AGE breaker is selected from L-carnosine, 3-phenacyl-4,5-dimethylthiazolium chloride, N-phenacylthiazolium bromide, 4,5-dimethylthiazolium bromide, and combinations thereof.
23. The formulation of para. 22, wherein the AGE breaker is L-carnosine.
24. The formulation of para. 1, wherein the anti-AGE agent is selected from glycation inhibitors and AGE formation inhibitors.
25. The formulation of para. 24, wherein the anti-AGE agent is selected from aminoguanidine, 4-(2,4,6-trichlorophenylureido)phenoxyisobutyric acid, 4-[(3,4-dichlorophenylmethyl)2-chlorophenylureido]phenoxyisobutyric acid, N,N'-bis(2-chloro-4-carboxyphenyl)formamidine, and combinations thereof.
26. The formulation of para. 1, further comprising a microcirculatory enhancer.
27. The formulation of para. 26, wherein the microcirculatory enhancer is phosphodiesterase inhibitor.
28. The formulation of para. 27, wherein the phosphodiesterase inhibitor is a Type (I) phosphodiesterase inhibitor.
29. The formulation of para. 28, wherein the phosphodiesterase inhibitor is vinpocetine.
30. The formulation of para. 1, further including at least one additive selected from thickeners, isotonic agents, and buffering agents.
31. The formulation of para. 1, having a pH in the range of about 6.5 to about 8.0.
32. The formulation of para. 32, having a pH in the range of about 6.8 to about 7.8.
33. A sterile ophthalmic formulation, comprising:
   a biocompatible chelating agent at a concentration of at least 0.6% by weight;
   an effective permeation-enhancing amount of methylsulfonylmethane; and
   a pharmaceutically acceptable ophthalmic carrier.
34. The formulation of para. 33, wherein the carrier is distilled or deionized water.
35. The formulation of para. 34, wherein the biocompatible chelating agent is selected from EDTA and ophthalmologically acceptable salts thereof.
36. The formulation of para. 35, wherein the biocompatible chelating agent represents up to 10 wt.% of the formulation.
37. The formulation of para. 33, wherein the methylsulfonylmethane represents approximately 1.0 wt.% to 33 wt.% of the formulation.
38. The formulation of para. 37, further comprising approximately 0.5 wt.% to 30 wt.% L-carnosine.
39. The formulation of para. 37, further comprising approximately 0.1 wt.% to 0.5 wt.% 3-phenacyl-4,5-dimethylthiazolium chloride.
40. The formulation of para. 37, further comprising approximately 1.0 wt.% to 2.0 wt.% dimethyl sulfoxide.
41. The formulation of para. 37, further comprising approximately 0.01 wt.% to 0.2 wt.% vinpocetine.
42. The formulation of para. 33, further including at least one additive selected from thickeners, isotonic agents, and buffering agents.
43. A sterile ophthalmic formulation, comprising:
   a biocompatible chelating agent at a concentration of at least 0.6% by weight;
   an effective AGE-reducing concentration of L-carnosine; and
   a pharmaceutically acceptable ophthalmic carrier.
44. The formulation of para. 43, wherein the carrier is distilled or deionized water.
45. The formulation of para. 44, wherein the biocompatible chelating agent is selected from EDTA and ophthalmologically acceptable salts thereof.
46. The formulation of para. 45, wherein the biocompatible chelating agent represents up to 10 wt.% of the formulation.
47. The formulation of para. 46, wherein the effective AGE-reducing concentration of L-carnosine is in the range of approximately 0.5% to 30% by weight.
48. The formulation of para. 43, further comprising approximately 0.01 wt.% to 0.2 wt.% vinpocetine.
49. The formulation of para. 43, further including at least one additive selected from thickeners, isotonic agents, and buffering agents.
50. A sterile ocular insert for delivery of an ophthalmic formulation to the eye, comprising a controlled release implant housing the formulation of any one of para.s 1, 33, and 43 and suitable for implantation into the conjunctiva, sclera, pars plana, anterior segment or the posterior segment of the eye.
51. The ocular insert of para. 50, wherein the implant is comprised of a polymeric matrix that gradually releases the formulation to the eye through diffusion and/or matrix degradation.
52. The ocular insert of para. 51, wherein the polymeric matrix is completely biodegradable.
53. The ocular insert of para. 50, wherein the implant is comprised of a laminated structure in which an inner core housing the formulation is contained between outer layers of a permeable polymer through which the formulation gradually diffuses.
54. A sterile ocular insert for delivery of an ophthalmic formulation to the eye, comprising a controlled release implant housing the formulation of any one of para.s 1, 33, and 43 and suitable for implantation into the conjunctiva, sclera, pars plana, anterior segment, or posterior segment of the eye.
55. The ocular insert of para. 54, wherein the implant is comprised of a polymeric matrix that gradually releases the formulation to the eye through dissolution of the matrix and/or diffusion.
56. The ocular insert of para. 55, wherein the polymeric matrix is completely soluble and/or biodegradable in the eye.
57. The ocular insert of para. 56, wherein the implant is comprised of a reservoir housing the formulation and enclosed in a polymeric membrane through which the formulation gradually diffuses.
58. The ocular insert of para. 55, wherein the implant is comprised of an osmotic system from which the formulation is gradually released as a result of increased osmotic pressure within the system following implantation in the eye.
59. A method for preventing or treating a mammalian individual susceptible to or afflicted with an adverse ocular condition, comprising topically administering the formulation of any one of para.s 1, 33, and 43 to an eye of the individual.
60. The method of para. 59, wherein the adverse ocular condition is associated with oxidative and/or free radical damage to the eye.
61. The method of para. 59, wherein the adverse ocular condition is a condition, disease, or disorder of the cornea, retina, lens, sclera, anterior segment, or posterior segment of the eye.
62. The method of para. 59, wherein the adverse ocular condition is associated with aging.
63. The method of para. 62, wherein the adverse ocular condition is opacification.
64. The method of para. 62, wherein the adverse ocular condition is decreased lens accommodation.
65. The method of para. 62, wherein the adverse ocular condition involves the formation of lipid deposits.
66. The method of para. 62, wherein the adverse ocular condition is visual acuity impairment.
67. The method of para. 62, wherein the adverse ocular condition is decreased contrast sensitivity.
68. The method of para. 62, wherein the adverse ocular condition is photophobia.
69. The method of para. 62, wherein the adverse ocular condition involves a decreased amount of light reaching the retina.
70. The method of para. 62, wherein the adverse ocular condition involves decreased pupil dilation.
71. The method of para. 62, wherein the adverse ocular condition is presbyopia.
72. The method of para. 62, wherein the adverse ocular condition is cataract formation.
73. The method of para. 72, wherein the adverse ocular condition is secondary cataract formation.
74. The method of para. 62, wherein the adverse ocular condition is age-related macular degeneration.
75. The method of para. 62, wherein the adverse ocular condition is elevated intraocular pressure.
76. The method of para. 62, wherein the adverse ocular condition is macular edema or macular scarring.
77. The method of para. 62, wherein the adverse ocular condition is band keratopathy.
78. The method of para. 62, wherein the adverse ocular condition comprises the presence of floaters in the vitreous humor.
79. The method of para. 62, wherein the adverse ocular condition is arcus senilis.
80. The method of para. 62, wherein the adverse ocular condition is dry eye syndrome.
81. The method of para. 59, wherein the adverse ocular condition comprises an ocular surface growth.
82. The method of para. 81, wherein the ocular surface growth is selected from pingueculae and pterygia.
83. The method of para. 59, wherein the adverse ocular condition is keratoconus.
84. A method for improving the visual acuity of a mammalian individual, comprising administering the formulation of any one of para.s 1, 33, and 43 to the eye of the individual.
85. A sterile ocular insert for administration of a biocompatible chelating agent to the eye, comprising a controlled release implant housing a formulation consisting essentially of the biocompatible chelating agent and a pharmaceutically acceptable carrier.
86. The insert of para. 85, wherein the biocompatible chelating agent is selected from EDTA and ophthalmologically acceptable salts thereof.
87. A sterile ocular insert for administration of an anti-AGE agent to the eye, comprising a controlled release implant housing a formulation consisting essentially of the anti-AGE agent and a pharmaceutically acceptable carrier.
88. The insert of para. 87, wherein the anti-AGE agent is L-carnosine.
89. The ocular insert of any one of para.s 84, 85, 86, or 87, wherein the implant is comprised of a polymeric matrix that gradually releases the formulation to the eye through dissolution of the matrix and/or diffusion.
90. The ocular insert of para. 89, wherein the polymeric matrix is completely soluble and/or biodegradable in the eye.
91. The ocular insert of any one of para.s 84, 85, 86, and 87, wherein the implant is comprised of a reservoir housing the formulation and enclosed in a polymeric membrane through which the formulation gradually diffuses.
92. The ocular insert of para. 91, wherein the implant is comprised of an osmotic system from which the formulation is gradually released as a result of increased osmotic pressure within the system following implantation in the eye.

## Claims

1. A sterile ophthalmic formulation, comprising:
a biocompatible chelating agent;
an effective permeation-enhancing amount of methylsulfonylmethane; and
a pharmaceutically acceptable ophthalmic carrier.

2. The formulation of claim 1, wherein the carrier is distilled or deionized water.

3. The formulation of claim 1 or 2, wherein the biocompatible chelating agent is selected from EDTA and ophthalmologically acceptable salts thereof.

4. The formulation of any preceding claim, wherein the biocompatible chelating agent represents up to 10 wt.% of the formulation.

5. The formulation of any preceding claim, wherein the methylsulfonylmethane represents approximately 1.0 wt.% to 33 wt% of the formulation.

6. The formulation of any preceding claim, further comprising approximately 0.5 wt.% to 30 wt.% L-carnosine.

7. The formulation of any preceding claim, further comprising approximately 0.1 wt.% to 0.5 wt.% 3-phenacyl-4,5-dimethylthiazolium chloride.

8. The formulation of any preceding claim, further comprising approximately 1.0 wt.% to 2.0 wt.% dimethyl sulfoxide.

9. The formulation of any preceding claim, further comprising approximately 0.01 wt.% to 0.2 wt.% vinpocetine.

10. The formulation of any preceding claim, further including at least one additive selected from thickeners, isotonic agents, and buffering agents.

11. The formulation of any preceding claim, wherein the biocompatible chelating agent is present at a concentration of at least 0.6% by weight.

12. A sterile ocular insert for delivery of an ophthalmic formulation to the eye, comprising a controlled release implant housing the formulation of any preceding claim and suitable for implantation into the conjunctiva, sclera, pars plana, anterior segment or the posterior segment of the eye.

13. A sterile ocular insert for administration of a biocompatible chelating agent or an anti-AGE agent to the eye, comprising a controlled release implant housing a formulation consisting essentially of the biocompatible chelating agent or anti-AGE agent and a pharmaceutically acceptable carrier.

14. The insert of claim 12, wherein the biocompatible chelating agent is selected from EDTA and ophthalmologically acceptable salts thereof.

15. The insert of claim 12 to 14, wherein the anti-AGE agent is L-carnosine.

16. The ocular insert of any one of claims 12 to 15, wherein the implant is comprised of a polymeric matrix that gradually releases the formulation to the eye through dissolution of the matrix and/or diffusion.

17. The ocular insert of claim 16, wherein the polymeric matrix is completely soluble and/or biodegradable in the eye.

18. The ocular insert of any one of claims 12 to 17, wherein the implant is comprised of a reservoir housing the formulation and enclosed in a polymeric membrane through which the formulation gradually diffuses.

19. The ocular insert of claim 18, wherein the implant is comprised of an osmotic system from which the formulation is gradually released as a result of increased osmotic pressure within the system following implantation in the eye.

20. The ocular insert of any one of claims 12 to 19, wherein the implant is comprised of a laminated structure in which an inner core housing the formulation is contained between outer layers of a permeable polymer through which the formulation gradually diffuses.

21. The ocular insert of any one of claims 12 to 20, wherein the implant is comprised of an osmotic system from which the formulation is gradually released as a result of increased osmotic pressure within the system following implantation in the eye.

22. Use of a biocompatible chelating agent for the preparation of a formulation of any one of claims 1 to 11 for the treatment or prevention of an adverse ocular condition in a mammalian individual susceptible to or afflicted that condition, wherein the formulation is administered topically to an eye of the individual.
